# EUROPEAN PATENT APPLICATION

(11) **EP 2 708 193 A1**
(43) Date of publication of application: **19.03.2014**
(21) Application number: 13181224.0
(22) Date of filing: 21.08.2013
(51) Int. Cl.: A61B 17/16

(54) **Bone preparation instrument**

(30) Priority: 14.09.2012 US 201261701066 P; 15.03.2013 US 201313832624
(71) Applicant: DePuy Synthes Products, LLC, Raynham, MA 02767-0350 (US)
(72) Inventor: HUFF, Daniel N, Warsaw, Indiana 46581 (US); GORAB, Robert S, Warsaw, Indiana 46581 (US); WINDHAGER, Reinhard, Warsaw, Indiana 46581 (US)
(74) Representative: Belcher, Simon James

(57) **Abstract**

An instrumentf or preparing bone, such as a femoral broach (10), includes a medial side (20) having a plurality of extraction teeth (34) extending over at least a portion of the medial side and an anterior side (22) having a plurality of compaction teeth (42) extending over at least a portion of the anterior side. The instrument further includes a lateral side (24) having a plurality of extraction teeth (59) extending over at least a portion of the lateral side and a posterior side (26) having a plurality of compaction teeth (60) extending over at least a portion of the posterior side. The medial side and lateral side also include a plurality of chip breakers (41) extending over at least one of the plurality of extraction teeth, the chip breakers sized and shaped so as to evacuate bone from the instrument. A system of bone instruments comprises at least two such broaches.

## Description

The present invention relates to an instrument for preparing a bone, and, more particularly, to improved instrument for broaching a cavity in bone for receiving a prosthesis.

For implantation of prosthetic stems, such as hip stems, accurate preparation of the bone or intramedullary canal is important in order to guarantee good contact between the prosthesis stem and the bone. The underlying concept behind precise preparation is that a precise bone envelope reduces the gaps between the stem and the bone, thereby improving the initial and long-term bone ingrowth/fixation. The bone canal is presently prepared for implantation of a prosthetic stem by drilling and reaming a resected end of a bone, such as a femur, and then preparing an area adjacent the drilled hole to provide a seat for the prosthetic stem or a proximal sleeve coupled to the stem of a modular prosthetic system. A sleeve of a modular prosthesis system is disclosed in US-5540694.

Preparation of the bone cavity can be achieved using tools such as reamers and broaches. A reamer is a rotating tool which can be used to create a cavity with a circular cross-section, which will frequently be constant along its length. A broach, such as that disclosed in US-5089004, is generally manipulated in an axial direction. It can be used to create a cavity with a non-circular cross-section. It is suitable for creation of the portion of a medullary cavity that is to receive the tapered portion of a prosthesis, in particular to ensure that the tapered shape of the prosthesis, which can be a complicated irregular shape, is properly matched by the shape of the internal surface of the bone cavity.

Proximally porous coated femoral hip stems, including the traditional "fit and fill" stem, have the intention to achieve contact with the cortices of the proximal femur for stable fixation. As such, they use broaches with "extraction" teeth which remove cancellous bone. However, there are regions of the proximal stem where cortical contact is simply not possible without a patient-specific or well fitting anatomic stem. In these regions extraction teeth have the capacity to remove chunks of cancellous bone leaving a gap surrounding the stem. There may be benefit to simply packing cancellous bone in those areas (done by "compaction teeth"), while allowing extraction teeth to achieve cortical contact in other regions.

The present invention provides a broach that includes both extraction teeth for removing the cancellous bone and compaction teeth for packing cancellous bone.

Accordingly, the invention provides an instrument for preparing bone includes a medial side having a plurality of extraction teeth extending over at least a portion of the medial side. The instrument further includes an anterior side having a plurality of compaction teeth extending over at least a portion of the anterior side and a lateral side having a plurality of extraction teeth extending over at least a portion of the lateral side. A posterior side is also included and has a plurality of compaction teeth extending over at least a portion of the anterior side. The medial side and lateral side also include a plurality of chip breakers extending over at least one of the plurality of extraction teeth, the chip breakers sized and shaped so as to evacuate bone from the instrument.

The invention also provides a femoral broach for preparing a femur for an implant includes a medial side having a plurality of extraction teeth extending over at least a portion of the medial side. The broach also includes an anterior side having a plurality of compaction teeth extending over at least a portion of the anterior side and a lateral side having a plurality of extraction teeth extending over at least a portion of the lateral side. A posterior side having a plurality of compaction teeth extending over at least a portion of the anterior side is also included. At least one of the medial side, anterior side, lateral side, and posterior side include a smooth portion at a distal end.

The invention also provides a system of bone instruments for preparing bone to receive an implant, the system comprising a plurality of broaches, at least two of the plurality of broaches includes a medial side having a plurality of extraction teeth extending over at least a portion of the medial side. At least two of the broaches also include an anterior side having a plurality of compaction teeth extending over at least a portion of the anterior side and a lateral side having a plurality of extraction teeth extending over at least a portion of the lateral side. A posterior side having a plurality of compaction teeth extending over at least a portion of the anterior side is also included in at least two of the broaches. The medial side and lateral side also include a plurality of chip breakers extending over at least one of the plurality of extraction teeth, the chip breakers sized and shaped so as to evacuate bone from the instrument.

The invention is described below by way of example with reference to the accompanying drawings, in which:
FIG. 1 is a partial cross-sectional view of a broach in a femoral bone.
FIG. 2 is a view of the medial side of the broach of FIG. 1.
FIG. 3 is a view of the anterior side of the broach of FIG. 1.
FIG. 4 is a view of the lateral side of the broach of FIG. 1.
FIG. 5 is a view of the posterior side of the broach of FIG. 1.
FIG. 6 is a close-up view of the broach of FIG. 1, on the section line A-A in FIG. 4.
FIG. 7 is a close-up view of the broach of FIG. 1, on the section line B-B.

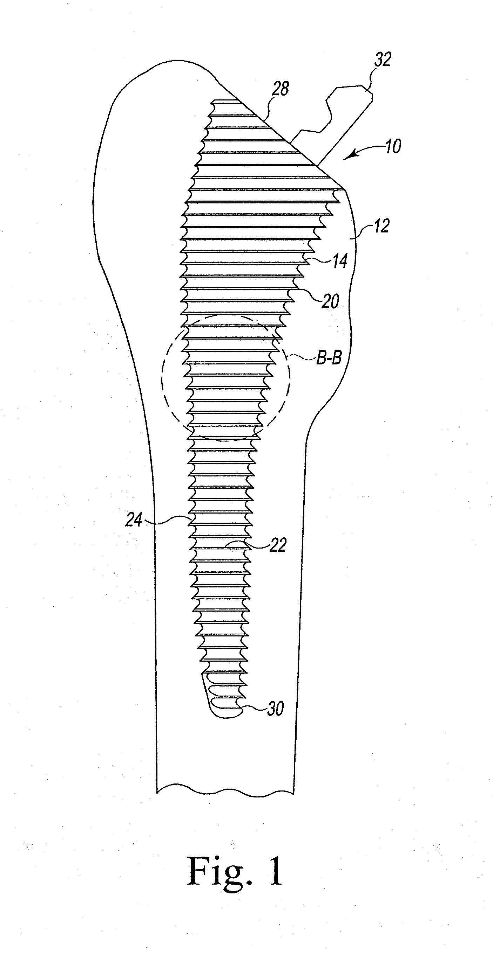

Referring to the drawings, FIG. 1 shows a femoral broach 10 positioned in a femur 12. The head of the femur 12 has been resected from a proximal end of the femur. The broach 10 is inserted into an intramedullary canal 14 of the femur 12. Cancellous bone, which is softer and somewhat spongy, surrounds the intramedullary canal. Surrounding the cancellous bone is cortical bone, which is stronger. As will be described in more detail below, the broach 10, is used to cut through portions of the cancellous and cortical bone to create a size-specific opening for a hip implant.

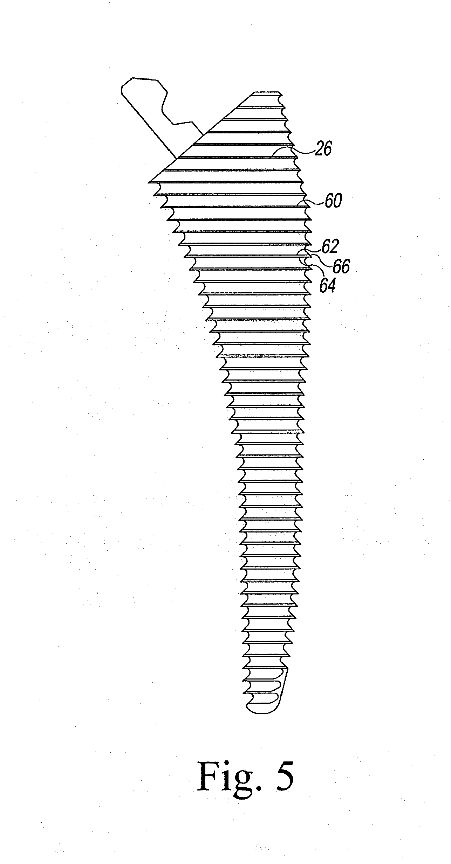

The broach includes a medial side 20, a lateral side 24, an anterior side 22, and a posterior side 26 (FIG. 5). The broach also includes a proximal portion 28 and a distal portion 30. Located at the top of the proximal portion 28 is a handle connection 32. The handle connection 32 allows the broach 10 to be coupled to a handle (not shown) or driver (not shown).

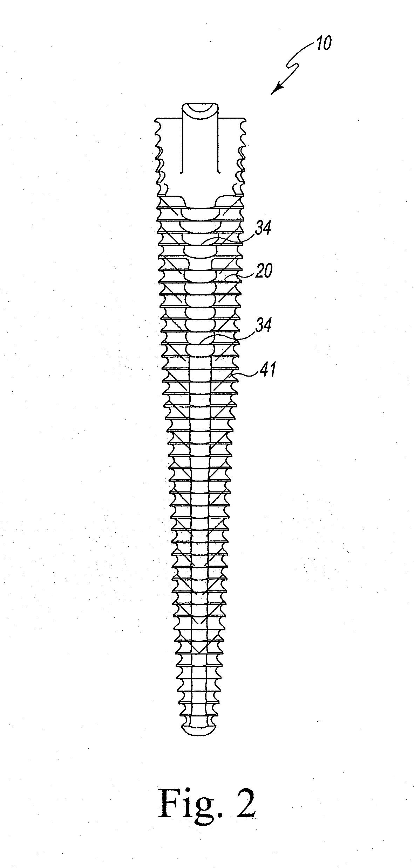

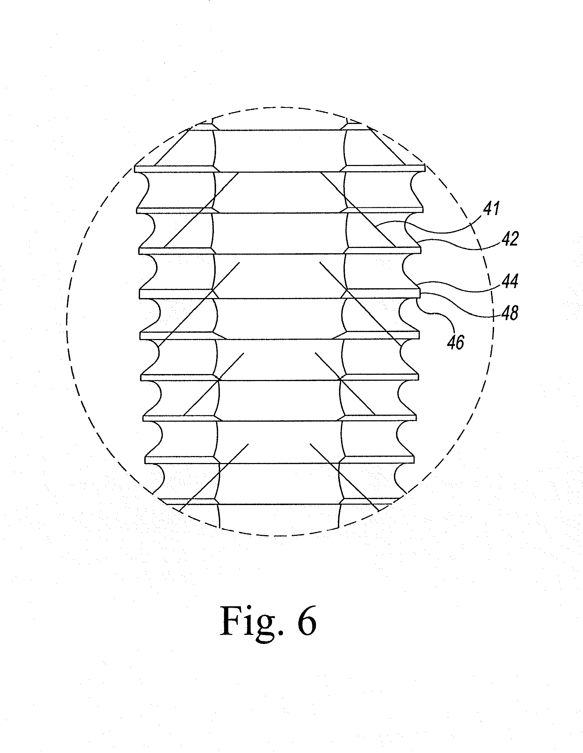

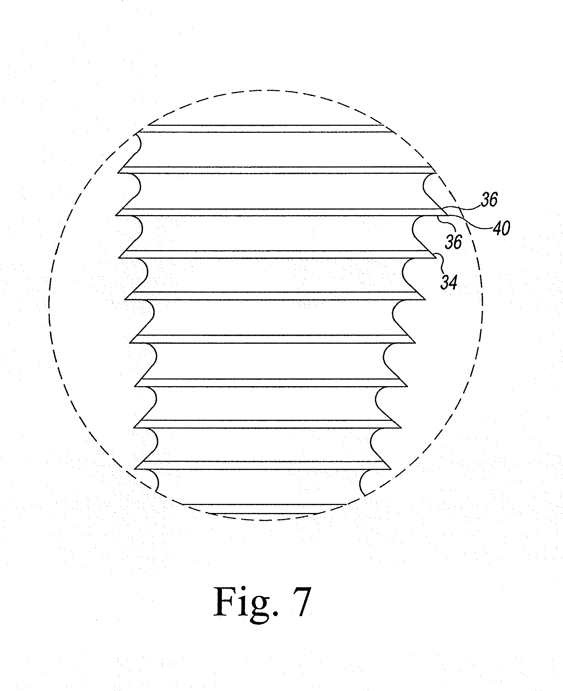

FIG. 2 shows the medial side 20 of the broach 10, including a plurality of teeth 34. The plurality of teeth 34 are made from two extending portions 36, 38 that meet at point 40 (FIG. 7). Because of the sharp points 40, the teeth cut into bone and are known as "extraction teeth". As shown in FIGS. 2 and 6, there are a plurality of chip breakers 41. The chip breakers 41 are located along the extraction teeth 34 and mainly aid in evacuating bone debris from the teeth. Optionally, the chip breakers 41 may extend over all of the extraction teeth 34. It is also envisaged that the chip breakers 41 might only extend over a part of the extraction teeth 34, allowing for adequate bone removal. In the instrument shown in FIG. 2, the extraction teeth 34 extend the whole length of the medial side 20. It is also envisaged that the extraction teeth 34 may only extend down a portion of the medial side 20. Because of the shape of the broach 10 (and subsequently implant), it is necessary that the cortical bone in the medial side be reached. The extraction teeth 34 cut through the cancellous bone and allow the cortical bone to reach. This allows for medial contact of the implant with hard cortical bone.

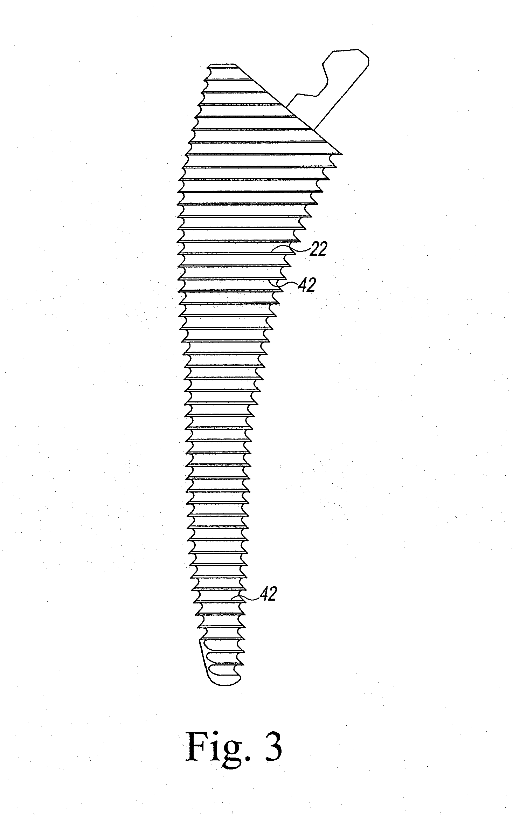

FIG. 3 shows the anterior side 22 of the broach 10, having a plurality of teeth 42 located on the anterior side 22. As seen in FIG. 6, the plurality of teeth 42 are made by two extending portion 44, 46 that join at a planar portion 48. In the instrument shown in FIG. 3, the planar portion 48 is a straight line edge. In other words, the extending portions 44, 46 do not meet at a point like the extending portions 36, 38 of the medial side 20. It is also envisaged that the two extending portions 44, 46 may be joined by a curve and not a straight line edge. Because these teeth 42 do not have a sharp point, the teeth 42 are known as "compaction teeth". Instead of cutting into the bone, the smooth, curved, or straight edges compact the bone. This is useful in areas of cancellous bone where cortical bone contact will largely not be achieved. Because of the shape of the stem used, much of the anterior side is left as cancellous, hence the compaction teeth. Therefore, the compaction teeth 42 are useful in creating the opening for the implant. In the instrument shown in FIG. 3, the compaction teeth 42 extend the entire length of the anterior side 22. It is also envisaged that the compaction teeth 42 may extend only over a portion of the anterior side 22.

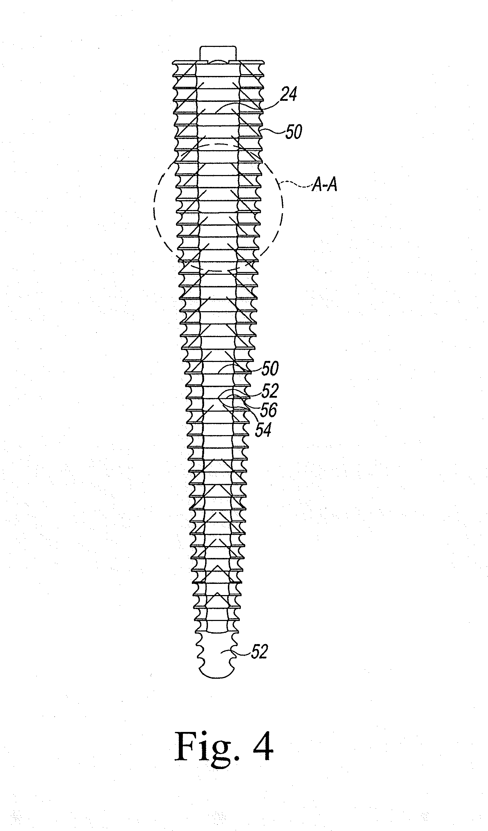

FIG. 4 shows the lateral side 24 of the broach 10. Similar to the medial side 20, the lateral side 24 includes a plurality of extraction teeth 50. The extraction teeth 50 are also made of two extending portions 52, 54 that converge at a point 56. As with the teeth 34 of medial side 20, the extraction teeth 50 of the lateral side 24 are used to extract cortical bone. In the instrument shown in FIG. 4, the extraction teeth 50 extend along most of the lateral side 24. At the distal portion 30 of the broach 10, there is a smooth portion 58 to reduce the risk of cortical perforation and to also act as a pilot to guide the broach down the canal without catching on teeth. It is also envisaged that extraction teeth 50 may extend the entire length of the lateral side 24. Optionally, the smooth portion 58 is between approximately 5 mm and 15 mm in length. Optionally, the smooth portion 58 will be on all sides, not just the lateral side 24 as shown in FIG. 4

FIG. 5 shows the posterior side 26 of the broach 10. Similar to the anterior side 22, the posterior side 26 has a plurality of compaction teeth 60. The compaction teeth 60 are made by two extending portion 62, 64 that join at a planar portion 66. In the instrument shown in FIG. 5, the planar portion 66 is a straight line edge. In other words, the extending portions 62, 64 do not meet at a point like the extending portions 36, 38 of the medial side 20. It is also envisaged that the two extending portions 62, 64 may be joined by a curve and not a straight line edge. Because the posterior side has more cancellous bone than cortical bone, the compaction teeth 60 are useful in creating the opening for the implant. In the instrument shown in FIG. 5, the compaction teeth 60 extend the entire length of the posterior side 26. It is also envisaged that the compaction teeth 60 may extend only over a portion of the posterior side 26.

The design of the broach 10 has numerous benefits. The broach 10 is less likely to remove bone that does not need to be removed to insert the implant. Also, it aids in preserving cancellous bone in areas where cortical contact is not possible, while still allowing cortical contact where such cortical bone is available.

Optionally, the broaches are made entirely from stainless steel. Alternatively, other metals may be used. Optionally, the broaches may be made with a hard plastic.

Optionally, a system having a plurality of broaches 10 is included. At least two of the plurality of broaches 10 have the same tooth configuration in that the medial side and lateral side include extraction teeth 34, 50 and the anterior side and posterior side have compaction teeth 42, 60 (the exact configuration and number of teeth may vary). Also, the sizes of the plurality of broaches may vary. This allows a surgeon to progressively broach, meaning that the surgeon may first use a small broach and then progressively use larger and larger broaches. Some of the broaches in the system may not have the same tooth configuration. For example, some of the broaches may only have compaction teeth while other broaches may have extraction teeth.

## Claims

1. An instrument for preparing bone, the instrument comprising:
a medial side having a plurality of extraction teeth extending over at least a portion of the medial side,
an anterior side having a plurality of compaction teeth extending over at least a portion of the anterior side,
a lateral side having a plurality of extraction teeth extending over at least a portion of the lateral side, and
a posterior side having a plurality of compaction teeth extending over at least a portion of the anterior side,
in which the medial side and lateral side also include a plurality of chip breakers extending over at least one of the plurality of extraction teeth, the chip breakers sized and shaped so as to evacuate bone from the instrument.

2. The instrument of claim 1, in which the instrument includes a proximal portion and a distal portion.

3. The instrument of claim 2, in which the distal portion of the lateral side includes a smooth portion.

4. The instrument of claim 2, in which the distal portion of at least one of the medial side, anterior side, lateral side, and posterior side includes a smooth portion.

5. The instrument of claim 2, in which the proximal portion includes a handle connection adapted to be coupled to a handle.

6. The instrument of claim 1, in which the extraction teeth of the medial side and the lateral side comprise of two extending portions that join at a point.

7. The instrument of claim 1, in which the compaction teeth of the anterior side and posterior side comprise two extending portions that join at a planar portion.

8. The instrument of claim 7, in which the planar portion is a straight line edge or is a curve.

9. A femoral broach for preparing a femur for an implant, the broach comprising:
a medial side having a plurality of extraction teeth extending over at least a portion of the medial side,
an anterior side having a plurality of compaction teeth extending over at least a portion of the anterior side,
a lateral side having a plurality of extraction teeth extending over at least a portion of the lateral side, and
a posterior side having a plurality of compaction teeth extending over at least a portion of the anterior side,
in which at least one of the medial side, anterior side, lateral side, and posterior side include a smooth portion at a distal end.

10. The femoral broach of claim 9, further comprising a proximal portion and the proximal portion includes a handle connection adapted to be coupled to a handle.

11. The femoral broach of claim 9, in which the extraction teeth of the medial side and the lateral side comprise of two extending portions that join at a point.

12. The femoral broach of claim 9, in which the compaction teeth of the anterior side and posterior side comprise two extending portions that join at a planar portion.

13. The femoral broach of claim 12, in which the planar portion is a straight line edge or is a curve.

14. A system of bone instruments for preparing bone to receive an implant, the system comprising at least two broaches, each as claimed in any one of claims 1 to 8.
